(19) Europäisches Patentamt
European Patent Office
Office européen des brevets

(11) Veröffentlichungsnummer : **0 423 694 B1**

(12) # EUROPÄISCHE PATENTSCHRIFT

(45) Veröffentlichungstag der Patentschrift : **02.02.94 Patentblatt 94/05**

(51) Int. Cl.$^5$ : **C07C 5/333,** B01J 35/02, C07C 15/46

(21) Anmeldenummer : **90119768.1**

(22) Anmeldetag : **15.10.90**

(54) **Verfahren zur katalytischen Gasphasen-Dehydrierung von Kohlenwasserstoffen, insbesondere von Alkylaromaten.**

(30) Priorität : **20.10.89 DE 3935073**

(43) Veröffentlichungstag der Anmeldung : **24.04.91 Patentblatt 91/17**

(45) Bekanntmachung des Hinweises auf die Patenterteilung : **02.02.94 Patentblatt 94/05**

(84) Benannte Vertragsstaaten : **BE DE ES FR GB IT**

(56) Entgegenhaltungen :
**EP-A- 0 082 831**
**BE-A- 891 237**
**US-A- 4 652 687**

(73) Patentinhaber : **SÜD-CHEMIE AG**
**Lenbachplatz 6**
**D-80333 München (DE)**

(72) Erfinder : **Kremer, Hans-Joachim, Dr.,Dipl.-Chem.**
**Zugspitzstr. 7**
**W-8011-Poing (DE)**
Erfinder : **Dethy, Jacques Maurice, Dr., Dipl.-Chem.**
**Ave. de l'Oud, Kabelleke 10, Bte 22**
**B-1140-Brüssel (BE)**
Erfinder : **André Louis, Ing.**
**Chaussée de Beaumont 19**
**B-7000 Mons (BE)**

(74) Vertreter : **Reitzner, Bruno, Dr. et al**
**Patentanwälte Dipl.-Ing. R. Splanemann Dr. B. Reitzner, Dipl.-Ing. K. Baronetzky Tal 13**
**D-80331 München (DE)**

**EP 0 423 694 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur katalytischen Gasphasen-Dehydrierung von Kohlenwasserstoffen, insbesondere von Alkylaromaten.

Die katalytische Gasphasen-Dehydrierung von Kohlenwasserstoffen, wie Alkylaromaten, insbesondere von Ethylbenzol, aber auch von gesättigten oder einfach ungesättigten aliphatischen Kohlenwasserstoffen, wie Propan oder Buten, in Gegenwart von Wasserdampf bei erhöhter Temperatur (im allgemeinen im Temperaturbereich von 500 bis 700°C) ist ein technisch in großem Umfang ausgeübtes Verfahren. Bei dem Verfahren gibt es je nach Art der Wärmeführung eine isotherme und eine adiabatische Ausführungsform. Eine ausführliche Beschreibung der üblichen Verfahren zur Dehydrierung von Alkylaromaten findet sich in "Industrielle Aromatenchemie" von H.G. Frank und J.W. Stadelhofer, Springer-Verlag/Berlin - Heidelberg 1987, Seiten 142 - 147. Die für diese Verfahren verwendeten Katalysatoren sind Metalloxidkatalysatoren. Die wirksamsten Katalysatoren bestehen im wesentlichen aus Eisenoxid als Hauptkomponente, oxidbildenden Alkalisalzen sowie aus strukturstabilisierenden, aktivitäts- und selektivitätssteigernden Metallverbindungen. Derartige Katalysatoren sind beispielsweise in der EP-A-0 177 832 beschrieben. Die Katalysatoren werden üblicherweise in Form von gepreßten zylindrischen Strängen, selten als Tabletten, Kugeln oder Ringe eingesetzt. Der Teilchendurchmesser der Katalysator-Formkörper variiert gewöhnlich zwischen 1,5 und 12 mm. Bei isothermen Verfahren muß infolge des hohen Druckverlustes in den Röhren des Dehydrierreaktors die Teilchengröße mindestens 5 mm betragen. In den Radialreaktoren, die bei adiabatischen Verfahren eingesetzt werden, können in den kürzeren Festbettschüttungen im allgemeinen Katalysatorstränge mit einem Durchmesser von etwa 1,5 bis 5 mm, und einem Längen-/Durchmesser-Verhältnis von etwa 0,5 bis 3 verwendet werden.

Aus kinetischen Gründen ist eine möglichst kleine Katalysatorteilchengröße wünschenswert, damit bei der selektiven Dehydrierreaktion bei nur kurzen Verweilzeiten im Katalysatorkörper, d.h. bei kurzen Diffusionswegen der Edukte und Produkte zur aktiven Katalysatoroberfläche und zurück in die Gasphase, maximale Ausbeuten an gewünschten Dehydrierprodukten erhalten werden.

Kleine Teilchenabmessungen verursachen jedoch einen hohen Druckverlust. Aus thermodynamischen Gründen ist für die Dehydrierreaktion ein möglichst niedriger Druck wünschenswert.

Nach der DE-AS 25 44 185 und der EP-B-0 206 192, die Verfahren zur katalytischen Dehydrierung von Alkylaromaten, insbesondere von Äthylbenzol, zum Gegenstand haben, wird diesem Anspruch durch Wahl besonderer Katalysatorgeometrien, z.B. Zylinderring, Stern- bzw. Kreuzstrang und Wabenform Rechnung getragen.

Wenngleich bei Verwendung derart geformter Katalysatorkörper eine erhöhte katalytische Wirkung gegenüber Zylindersträngen erzielt wurde, bleibt aufgrund der hohen technischen Bedeutung von Verfahren zur katalytischen Dehydrierung von Kohlenwasserstoffen, insbesondere zur Herstellung von Styrol, die Aufgabe einer weiteren Verbesserung der Aktivität und Selektivität der Dehydrierkatalysatoren durch kinetisch günstigere Formgebung bestehen.

Aus der DE-A-31 41 942 sind Katalysatorformkörper von zylindrischer Gestalt mit mehreren Längsvertiefungen bekannt, die sich radial vom Zylinderumfang nach innen erstrecken und dazwischenliegende Erhebungen begrenzen, deren maximale Breite größer ist als diejenige der Vertiefungen. Diese Katalysatorformkörper werden zur Umwandlung von Kohlenwasserstoffen, insbesondere bei der Isomerisierung, Alkylierung, Reformierung und Hydrierung, einschließlich von Hydrocrackung, Hydrobehandlung, Hydroraffinierung, Hydrometallierung, Hydrodesulforierung und Hydrodenitrogenierung verwendet. Diese Reaktionen werden in der flüssigen Phase durchgeführt, weshalb hohe Rückhaltezeiten für Flüssigkeiten erwünscht sind, was durch den kleeblattförmigen Querschnitt in Verbindung mit dem verhältnismäßig geringen Durchmesser (< 0,23 cm) der Katalysatorformkörper begünstigt wird.

Ferner werden die genannten Flüssigphasen-Reaktionen bevorzugt mit Trägerkatalysatoren durchgeführt, wobei als Trägermaterial vorzugsweise Aluminiumoxid verwendet wird, auf das als katalytisch wirksame Substanzen Metalle, Metalloxide und Metallsulfide von Übergangselementen (z.B. von Nickel, Kobalt, Molybdän und/oder Wolfram) aufgebracht werden können. Derartige Katalysatoren sind für katalytische Gasphasen-Dehydrierungen von Kohlenwasserstoffen nicht geeignet, da sie infolge von Kohlenstoffabscheidungen in kurzer Zeit desaktiviert werden.

Aus der EP-A-0 082 831 sind zahnradförmige Vanadiumpentoxid-Katalysatoren für die Oxidation von Schwefeldioxid zu Schwefeltrioxid bekannt. Hinweise auf die katalytische Gasphasen-Dehydrierung von Kohlenwasserstoffen finden sich in dieser Druckschrift nicht.

Aus der US-A-4 652 687 ist ein Verfahren zu Dehydrierung von Kohlenwasserstoffen unter Verwendung von sternförmigen Katalysator-Formkörpern bekannt. Diese Formkörper unterscheiden sich von den erfindungsgemäß verwendeten Formkörpern grundlegend im Hinblick auf die Abmessungs-verhältnisse.

Es wurde nun gefunden, daß man bei einem Verfahren zur katalytischen Gasphasen-Dehydrierung von Kohlenwasserstoffen, insbesondere von Alkylaromaten, bei erhöhter Temperatur in Gegenwart von Wasserdampf und Metalloxidkatalysator-Formkörpern mit Eisenoxid als Hauptkomponente besonders vorteilhafte Ergebnisse erzielt, wenn man als Katalysatoren zahnradförmige Formkörper mit mindestens drei Zähnen, einem Kopfkreisdurchmesser ($d_2$) von 0,35 bis 0,6 cm und folgenden Abmessungsverhältnissen einsetzt:

(a) das Verhältnis Kopfkreisdurchmesser ($d_2$) : Fußkreisdurchmesser ($d_1$) beträgt etwa 1,2 bis 2,5 : 1;
(b) das Verhältnis Lückenbreite am Zahnfuß ($b_1$) : Zahnbreite an der Krone ($b_2$) beträgt 0,1 bis 0,9 : 1
(c) die Lückenbreite am Zahnfuß ($b_1$) beträgt mindestens 0,1 mm.

Die Begriffe "Kopfkreisdurchmesser, Fußkreisdurchmesser und Zahnhöhe" sind der Terminologie auf dem Gebiet der mechanischen Zahnräder entnommen (vgl. beispielsweise Friedrich, Tabellenbuch Metall- und Maschinentechnik, 1988, Seite 4-45).

Das erfindungsgemäß vorgesehene Verhältnis (a) zwischen Kopfkreisdurchmesser ($d_2$) und Fußkreisdurchmesser ($d_1$), das die Zahnhöhe (h) bestimmt, ist deshalb wichtig, um zu gewährleisten, daß bei einer Schüttung bzw. einer geordneten Anordnung der Katalysatorkörper im Reaktor die durch $b_2$ und die Länge des Formkörpers bestimmte Kronenfläche nicht auf der durch die Lückenbreite am Zahnfuß ($b_1$) und die Länge des Formkörpers bestimmte Fläche am Zahnfuß aufliegt.

Das erfindungsgemäß vorgesehene Verhältnis (b) zwischen Zahnhöhe (h) und Kopfkreisdurchmesser ($d_2$) ist einerseits wichtig, um ein Abbrechen der Zähne zu verhindern und andererseits, um den Flächenkontakt zwischen den einzelnen Formkörpern in einer Reaktorfüllung zu reduzieren.

Vorzugsweise setzt man Formkörper mit folgenden Abmessungsverhältnissen ein:

(a) das Verhältnis Kopfkreisdurchmesser ($d_2$) : Fußkreisdurchmesser ($d_1$) beträgt etwa 1,3 bis 1,6 : 1; vorzugsweise 1,5 : 1;
(b) das Verhältnis Lückenbreite am Zahnfuß ($b_1$) : Zahnbreite an der Krone beträgt etwa 0,2 bis 0,6 : 1;
(c) die Lückenbreite am Zahnfuß ($b_1$) beträgt mindestens 0,15 mm.

Der Katalysator besteht entweder aus Aktivmasse (Volkatalysator) oder ist mit Aktivmasse beschichtet (Trägerkatalysator). Vollkatalysatoren werden bevorzugt.

Weiterhin setzt man vorzugsweise zahnradförmige Formkörper mit jeweils 5 bis 8 Zähnen ein. Durch die Verwendung zahnradförmiger Formkörper mit einem Kopfkreisdurchmesser ($d_2$) von mindestens 0,35 cm wird der Druckverlust im Reaktor vermindert, insbesondere wenn die Dehydrierungsreaktionen bei vermindertem Druck durchgeführt werden. Auf der anderen Seite beträgt der Kopfkreisdurchmesser ($d_2$) nicht mehr als 0,6 cm, da die spezifische Oberfläche größerer Formkörper geringer ist, was zu einem Aktivitätsabfall führt.

Vorzugsweise setzt man zahnradförmige Formkörper ein, die einen oder mehrere Längskanäle, vorzugsweise innerhalb des Fußkreisdurchmessers ($d_1$) enthalten.

Nach einer weiteren bevorzugten Ausführungsform setzt man zahnradförmige Formkörper mit im wesentlichen parallelen Zahnflanken auf mindestens einem Teil der Zahnhöhe (h) ein. Auf diese Weise kann die Bruchgefahr vermindert werden, da zwischen Zahnkrone und Zahnflanke kein spitzer Winkel mehr vorhanden ist, wie es z.B. bei kleeblattförmigen Katalysatorformkörpern der Fall ist. Die Festigkeit der zahnradförmigen Formkörper kann weiterhin dadurch verbessert werden, daß deren Kanten an der Zahnkrone abgefast oder abgerundet sind.

Nach dem erfindungsgemäßen Verfahren wird vorzugsweise Ethylbenzol zu Styrol dehydriert.

Einige zahnradförmige Querschnitte der erfindungsgemäß verwendeten Formkörper sind in den Figuren 1 bis 3 dargestellt. Es handelt sich hierbei um Formkörper mit fünf abgeplatteten Zähnen, wobei der Formkörper von Fig. 1 keinen Längskanal hat, während der Formkörper nach Fig. 2 einen glatten Längskanal und der Formkörper nach Fig. 3 einen Längskanal mit zahnradförmigem Querschnitt aufweist.

Die einzelnen Abmessungen sind in Fig. 1 angegeben. Mit $d_1$ ist der Fußkreisdurchmesser, mit $d_2$ der Kopfkreisdurchmesser bezeichnet. Mit h ist die Zahnhöhe und mit $b_2$ die Zahnbreite an der Krone bezeichnet. Diese ist in Fig. 1 identisch mit der Breite am Zahnfuß, kann aber auch geringer sein, wenn der Zahn an den Kanten abgefast oder abgerundet ist, wie es in Fig. 3 dargestellt ist. Die Breite der Zahnlücke am Zahnfuß ist mit $b_1$ bezeichnet. Das Verhältnis zwischen Kopfkreisdurchmesser $d_2$ und Fußkreisdurchmesser $d_1$ beträgt bei der dargestellten Ausführungsform etwa 1,5 : 1, das Verhältnis zwischen Lückenbreite am Zahnfuß und Zahnbreite an der Krone etwa 0,27 : 1.

Die Länge der Formkörper wird im allgemeinen so bemessen, daß das Verhältnis zum Kopfkreisdurchmesser $d_2$ (der bei zylindrischen Formkörpern dem Außendurchmesser entspricht) etwa 0,5 bis 5 : 1, vorzugsweise 1 bis 3 : 1 beträgt. Der Kopfkreisdurchmesser beträgt im allgemeinen etwa 1,5 bis 12 mm, vorzugsweise 3 bis 9 mm. Die Zahnflanken verlaufen über die Zahnhöhe (h) im wesentlichen parallel, wie auch bei den anderen Ausführungsformen, d.h. die Zahnflanken stehen zu der Zahnkrone in einem rechten Winkel.

Bei der Ausführungsform nach Fig. 2 ist ein glatter, zylindrischer Längskanal mit dem Durchmesser $d_3$ vorgesehen. Dieser Längskanal hat bei der Ausführungsform von Fig. 3 einen zahnradförmigen Querschnitt mit einem Kopfkreisdurchmesser $d_4$ und einem Fußkreisdurchmesser $d_5$. Die anderen Abmessungen bzw. Abmessungsverhältnisse entsprechenden denen von Fig. 1.

Anhand von Fig. 3 ist ferner erläutert, daß die Zähne an der Kronenkante abgefast oder abgerundet sein können, wobei die Zahnbreite an der Krone ($b_2$) größer als die Lückenbreite am Zahnfuß ($b_1$) ist.

Die Abfasung bzw. Abrundung der Kanten an der Krone bei der Ausführungsform Fig. 3 hat ferner den Vorteil, daß sich die Zahnkronen bei einer Katalysatorschüttung bzw. einer geordneten Anordnung auf einer kleineren Fläche berühren, so daß insgesamt eine größere freiliegende Oberfläche zur Verfügung steht. Ein weiterer Vorteil der Abrundung bzw. Abfasung der Zähne besteht darin, daß der Abrieb des Katalysators vermindert wird, wenn dieser in den Reaktor eingefüllt wird bzw. wenn die Reaktorfüllung im Betrieb Vibrationen oder Temperatur- und Druckwechselbeanspruchungen, die zu einer Verschiebung der Formkörper relativ zueinander führen können, ausgesetzt ist.

Weitere Ausführungsformen der erfindungsgemäß verwendeten Formkörper sind in den Fig. 4 und 5 dargestellt, wobei Fig. 4 einen Formkörper mit 3 Zähnen und Fig. 5 einen Formkörper mit 6 Zähnen zeigt. Die einzelnen Abmessungen sind ebenfalls eingetragen.

Um den vorstehend genannten kinetischen Anforderungen an den Dehydrierkatalysator (kurze Diffusionswege in Verbindung mit geringen Druckverlusten) zu entsprechen, muß auf eine große geometrische Oberfläche pro Volumeneinheit in Verbindung mit einem geringen Druckverlust entlang der Schüttung der Katalysator-Formkörper geachtet werden. Ein Maß hierfür ist der Aktivitätsindex (AI), der sich aus der Gesamtfläche (geometrische Oberfläche) der Formkörper in einem Liter mit dem Druckverlust $\Delta p$, bezogen auf die Formkörperschüttung in einem Reaktor mit einer Länge von 1 m und einem Durchmesser von 1 m errechnet.

$$AI = \frac{\text{Fläche (cm}^2\text{)}}{\text{Volumen (cm}^3\text{)}} \cdot \frac{1}{\Delta p}$$

Der Druckverlust $\Delta p$ (in Pascal) errechnet sich aus der modifizierten Lewa-Gleichung

$$p = \frac{2{,}16 \cdot H \cdot G^{1{,}9}}{b' \cdot p \cdot \frac{273}{T} \cdot D_p^{\;1{,}1}},$$

worin H die Höhe der Schüttung (1 m), G die Querschnittsbelastung (kg Gas pro $m^2$, freie Reaktorfläche und sec.), b' die Dichte des Gases ($kg/m^3$), p der Druck des Gases am Eingang ($N/m^2$), T die Temperatur in K und $D_p$ den Äquivalentdurchmesser des Formkörpers (entsprechend einer Kugel mit gleichem Volumen) in m bedeuten. Für die Errechnung von p in Tabelle 1 wurden folgende Variablen konstant gehalten: H, G, b, p und T, so daß $\Delta p$ nur noch proportional zu $= \frac{1}{D_p}{}_{1,1}$ ist.

Als Gas wurde ein Ethylbenzol-Wassergemisch (Gewichtsverhältnis 1 : 2,0) mit einem Eingangsdruck p von Atmosphärendruck und einer Temperatur von 893 K verwendet.

Bezogen auf den gleichen äußeren Durchmesser (Kopfkreisdurchmesser) $d_2$ liegt der Aktivitätsindex bei Formkörpern ohne zentralen Längskanal um wenigstens 20 % über dem der glatten Zylinderstrangform. Bei Formkörpern mit zentralem Längskanal erhöht sich der Aktivitätsindex noch weiter.

In Tabelle I sind die Formkörperabmessungen und Aktivitätsindices für einige erfindungsgemäß verwendete Katalysator-Formkörper im Vergleich mit den bekannten Katalysator-Formkörpern zusammengestellt. Die Werte von Tabelle I zeigen, daß die erfindungsgemäß verwendeten Formkörper (Beispiele 3, 6, 10 und 11) sowohl gegenüber den glatten Vollstrangzylindern (Beispiele 1, 2, 4, 5, 9, 12) als auch gegenüber den sternförmigen Formkörpern nach der DE-AS 25 44 185 (Beispiele 7 und 8) sowohl niedrigere $\Delta p$-Werte als auch höhere Aktivitätsindices haben.

TABELLE I

| Beispiel | Außendurchmesser bzw. Kopfkreisdurchmesser $d_2$ (mm) | Länge (mm) | Längskanaldurchmesser d(mm) | Fußkreisdurchmesser bei gezahnt. Formkörpern $d_1$ (mm) | Verhältnis $\frac{d_2}{d_1}$ | Anzahl d. Zähne N | $\Delta p$ | AI | Bemerkungen |
|---|---|---|---|---|---|---|---|---|---|
| 1 | 3.0 | 6.0 | -- | - | - | 0 | 1.55 | 6380 | Vergleich |
| 2 | 3.5 | 6.0 | -- | - | - | 0 | 1.35 | 6460 | " |
| 3 | 3.5 | 6.0 | -- | 2.5 | 1.4 | 5 | 1.17 | 8690 | Fig. 1 |
| 4 | 4.0 | 7.0 | -- | 4.0 | - | 0 | 1.16 | 6560 | Vergleich |
| 5 | 4.5 | 7.0 | -- | - | - | 0 | 1.05 | 6630 | " |
| 6 | 4.5 | 7.0 | -- | 3.2 | 1.4 | 5 | 0.90 | 8960 | Fig. 1 |
| 7 | 4.5 | 7.0 | -- | 2,68 | 1.74 | 6 | 1.25 | 8840 | Fig.2 v.DAS2544185 |
| 8 | 4.5 | 7.0 | -- | 2,01 | 2.25 | 4 | 1.21 | 7980 | Fig.3 v.DAS2544185 |
| 9 | 6.0 | 9.0 | -- | 0 | - | 0 | 0.77 | 6830 | Vergleich |
| 10 | 6.0 | 9.0 | -- | 4.30 | 1.4 | 5 | 0.66 | 9140 | Fig. 1 |
| 11 | 6.0 | 9.0 | 1.75 | 4.30 | 1.4 | 5 | 0.60 | 11990 | Fig. 2 |
| 12 | 9.0 | 13.5 | -- | 0 | - | 0 | 0.49 | 7160 | Vergleich |

Fig. 6 gibt den Aktivitätsindex als Funktion des Rohr-Reaktordurchmessers für Formkörper-Außendurch-

messer (Kopfkreisdurchmesser $d_2$) von 6 mm und einer Länge von 9 mm wieder. Kurve A zeigt die Aktivitätsindices von Katalysatoren mit zahnförmigem Querschnitt und zentralem Kanal entsprechend Fig. 2. Kurve B zeigt die Aktivitätsindices von Formkörpern mit zahnradförmigem Querschnitt ohne zentralen Längskanal gemäß Fig. 1. Kurve C zeigt die Aktivitätsindices von glatten Vollstrangzylindern. Die Kurven zeigen deutlich die Überlegenheit der erfindungsgemäß verwendeten Zahnradformen gemäß Fig. 2 bzw. 1 gegenüber dem glatten Vollstrangzylinder. Die Überlegenheit der erfindungsgemäß verwendeten Formkörper kommt sowohl in isothermen Rohrreaktoren als auch in adiabatischen, radial oder vertikal durchströmten Festbettreaktoren zur Geltung

Die Figuren 7 und 8 zeigen die relativen Aktivitätsindices für Formkörper-Außendurchmesser (Kopfkreisdurchmesser) von 3,5, 4,5 und 6 mm bei den erfindungsgemäß verwendeten Formkörpern in direktem Vergleich zu zylindrischen Vollstrang-Formkörpern. Der relative Aktivitätsindex dieser bekannten Formkörper wurde auf 100 % festgelegt. Neben dem relativen Aktivitätsindex (AI), der in der jeweils dritten Kolonne angegeben ist, sind auch die relativen spezifischen Oberflächen (5) (zweite Kolonne) und die relativen Druckunterschiede ($\Delta p$) (erste Kolonne) angegeben, wobei für die Vergleichsformkörper auch bei diesen Parametern jeweils 100 % angenommen wurde.

Daraus ergibt sich, daß die erfindungsgemäß verwendeten Formkörper bei geringeren $\Delta p$-Werten und höheren Oberflächen höhere Aktivitätsindices aufweisen.

Die überraschende Aktivität und Selektivität der gezahnten Formkörper ist anhand von Vergleichsversuchen entsprechend dem nachstehend angegebenen Anwendungsbeispiel erläutert.

ANWENDUNGSBEISPIEL

Die Vergleichskatalysatoren sowie die erfindungsgemäß verwendeten Katalysatoren mit verschiedenen Außendurchmessern wurden nach an sich bekannten Verfahren hergestellt und getestet. Ein solches Verfahren ist zum Beispiel in der EP-A 0 177 832, Beispiel 7, beschrieben.

Das Verfahren besteht im wesentlichen darin, daß ein Gemisch aus Eisen(III)-Oxid, Chrom(VI)-Oxid, Kaliumcarbonat, Magnesiumcarbonat und Calciumcarbonat mit Wasser vermischt und geknetet wird. Aus der erhaltenen extrudierbaren Paste werden Formkörper gepreßt, die getrocknet und 6 Stunden bei 540°C calciniert wurden. Der Katalysator hatte die folgende Zusammensetzung:

| | |
|---|---|
| $Fe_2O_3$ | 64,3 Gew.-% |
| $Cr_2O_3$ | 3,6 Gew.-% |
| $K_2O$ | 24,3 Gew.-% |
| MgO | 2,5 Gew.-% |
| CaO | 5,3 Gew.-%. |

Die Prüftemperatur wurde so gewählt, daß bei einem Dampf-Ethylbenzolverhältnis von 2,0 kg/kg, bei Normaldruck und einer Flüssigraumgeschwindigkeit von 1 Liter Ethylbenzol je Liter Katalysator und Stunde ein Ethylbenzolumsatz von 70 Gew.-% erzielt wurde. Die Ergebnisse sind in Fig. 9 graphisch dargestellt. Auf der linken Ordinate ist die Temperatur in °C aufgetragen, bei der ein Ethylbenzolumsatz von 70 Gew.-% erzielt wurde. Auf der rechten Ordinate ist die Selektivität (wiederum bei einem Ethylbenzolumsatz von 70 Gew.-%) in Prozent auf Gewichtsbasis angegeben. Auf der Abszisse sind die Teilchendurchmesser (Kopfkreisdurchmesser) in mm angegeben. Die Kurven A und B zeigen die mit den Vollstrangzylindern erhaltenen Temperatur- bzw. Selektivitätswerte, während die Kurven C und D die mit den erfindungsgemäß verwendeten gezahnten Formkörpern (gemäß Fig. 1) erhaltenen Temperatur- bzw. Selektivitätswerte als Funktion des Teilchendurchmessers zeigen. Die angegebenen Werte zeigen, daß mit den erfindungsgemäß verwendeten Formkörpern eine höhere Aktivität und Selektivität erzielbar ist.

## Patentansprüche

1. Verfahren zur katalytischen Gasphasen-Dehydrierung von Kohlenwasserstoffen, insbesondere von Alkylaromaten, bei erhöhter Temperatur in Gegenwart von Wasserdampf und Metalloxidkatalysator-Formkörpern mit Eisenoxid als Hauptkomponente, dadurch gekennzeichnet, daß man als Katalysatoren zahnradförmige Formkörper mit mindestens drei Zähnen, einem Kopfkreisdurchmesser ($d_2$) von 0,35 bis 0,6 cm und folgenden Abmessungsverhältnissen einsetzt:
   (a) das Verhältnis Kopfkreisdurchmesser ($d_2$): Fußkreisdurchmesser ($d_1$) beträgt etwa 1,2 bis 2,5 : 1;
   (b) das Verhältnis Lückenbreite am Zahnfuß ($b_1$): Zahnbreite an der Krone ($b_2$) beträgt 0,1 bis 0,9 : 1;
   (c) die Lückenbreite am Zahnfuß ($b_1$) beträgt mindestens 0,1 mm.

2. Verfahren nach Anspruch 1, dadurch gekennzeichnet, daß man als Katalysatoren zahnradförmige Formkörper mit folgenden Abmessungsverhältnissen einsetzt:
(a) das Verhältnis Kopfkreisdurchmesser ($d_2$): Fußkreisdurchmesser ($d_1$) beträgt etwa 1,3 bis 1,6 : 1; vorzugsweise 1,5:1;
(b) das Verhältnis Lückenbreite am Zahnfuß ($b_1$): Zahnbreite an der Krone ($b_2$) beträgt etwa 0,2 bis 0,6 : 1;
(c) die Lückenbreite am Zahnfuß ($b_1$) beträgt mindestens 0,15mm.

3. Verfahren nach Anspruch 1 oder 2, dadurch gekennzeichnet, daß man zahnradförmige Formkörper mit jeweils 5 bis 8 Zähnen einsetzt.

4. Verfahren nach einem der Ansprüche 1 bis 3, dadurch gekennzeichnet, daß man zahnradförmige Formkörper einsetzt, die einen oder mehrerer Längskanäle, vorzugsweise innerhalb des Fußkreisdurchmessers ($d_1$), enthalten.

5. Verfahren nach einem der Ansprüche 1 bis 4, dadurch gekennzeichnet, daß man zahnradförmige Formkörper mit im wesentlichen parallelen Zahnflanken auf mindestens einem Teil der Zahnhöhe (h) einsetzt.

6. Verfahren nach einen der Ansprüche 1 bis 5, dadurch gekennzeichnet, daß man zahnradförmige Formkörper deren Kanten an der Zahnkrone abgefaßt oder abgrundet sind, einsetzt.

7. Verfahren nach einem der Ansprüche 1 bis 6, dadurch gekennzeichnet, daß der Katalysator aus Aktivmasse besteht (Vollkatalysator) oder mit Aktivmasse beschichtet ist (Trägerkatalysator).

8. Verfahren nach einem der Ansprüche 1 bis 7, dadurch gekennzeichnet, daß Ethylbenzol zu Styrol dehydriert wird.

## Claims

1. A process for the gas phase catalytic dehydrogenation of hydrocarbons, in particular of aromatic alkyls at high temperature in the presence of water vapour and metal oxide catalyst moulded bodies with iron oxide as the main constituent, characterised in that as catalysts toothed-wheel shaped moulded bodies are used having at least three teeth, an outside diameter ($d_2$) of 0.35 to 0.6 cm and the following dimensional relationships:
(a) the ratio of outside diameter ($d_2$): root diameter ($d_1$) is approximately 1.2 to 2.5 : 1;
(b) the ratio of gap width at the tooth base ($b_1$): tooth width at the crown ($b_2$) is 0.1 to 0.9 : 1;
(c) the gap width at the tooth root ($b_1$) is at least 0.1 mm.

2. A process according to Claim 1, characterised in that as catalysts toothed-wheel shaped moulded bodies with the following dimensional relationships are used:
(a) the ratio of outside diameter ($d_2$): root diameter ($d_1$) is approximately 1.3 to 1.6 : 1, preferably 1.5 : 1;
(b) the ratio of gap width at the tooth base ($b_1$): tooth width at the crown ($b_2$) is approximately 0.2 to 0.6 : 1;
(c) the gap width at the tooth base ($b_1$) is at least 0.15 mm.

3. A process according to Claim 1 or 2, characterised in that toothed-wheel shaped moulded bodies each with 5 to 8 teeth are used.

4. A process according to any one of Claims 1 to 3, characterised in that toothed-wheel shaped moulded bodies are used which comprise one or more longitudinal channels, preferably within the root diameter ($d_1$).

5. A process according to any one of Claims 1 to 4, characterised in that toothed-wheel shaped moulded bodies are used having substantially parallel tooth flanks on at least portion of the tooth height (h).

6. A process according to any one of Claims 1 to 5, characterised in that toothed-wheel shaped moulded bodies are used, the edges of which are bevelled or rounded at the tooth crown.

7. A process according to any one of Claims 1 to 6, characterised in that the catalyst consists of active material (solid catalyst) or is coated with active material (supported catalyst).

8. A process according to any one of Claims 1 to 7, characterised in that ethyl benzene is dehydrogenated to styrene.

**Revendications**

1. Procédé de déshydrogénation catalytique en phase gazeuse des hydrocarbures, notamment des alkylaromatiques, à haute température en présence de vapeur d'eau et de pièces de forme de catalyseur à base d'oxyde métallique, utilisant de l'oxyde de fer comme composant principal, caractérisé en ce qu'on utilise comme catalyseur des pièces de forme en forme de roue dentée comprenant au moins trois dents, possédant un diamètre de cercle de tête ($d_2$) de 0,35 à 0,6 cm et possédant le rapport de dimension suivant :
   (a) le rapport diamètre de cercle de tête ($d_2$) : diamètre de cercle creux de denture ($d_1$ ) est d'environ 1,2 à 2,5 : 1;
   (b) le rapport largeur de creux de denture au pied de dent ($b_1$) : largeur des dents à la tête ($b_2$) est de 0,1 à 0,9 : 1;
   (c) la largeur de creux de denture au pied des dents ($b_1$) est d'au moins 0,1 mm.

2. Procédé selon la revendication 1, caractérisé en ce qu'on utilise, comme catalyseurs, des pièces de forme présentant les rapports de cotes suivants :
   (a) le rapport diamètre du cercle de tête $d_{(2)}$ : diamètre du cercle de pied ($d_1$) est d'environ 1,3 à 1,6 : 1, de préférence de 1,5 : 1 ;
   (b) le rapport largeur du creux de denture au pied de dent ($b_1$) : largeur de la dent à la tête est d'environ 0,2 à 0,6: 1 ;
   (c) la largeur du creux au pied de dent ($b_1$) est d'au moins 0,15 mm.

3. Procédé selon la revendication 1 ou 2, caractérisé en ce qu'on utilise des pièces de forme en forme de roue dentée possédant 5 à 8 dents.

4. Procédé selon l'une des revendications 1 à 3, caractérisé en ce qu'on utilise des pièces de forme en forme de roue dentée qui présentent un ou plusieurs canaux longitudinaux, de préférence à l'interieur du diamètre du cercle de pied ($d_1$).

5. Procédé selon une des revendications 1 à 4, caractérisé en ce qu'on utilise des pièces de forme en forme de roue dentée qui possèdent des flancs de dents sensiblement parallèles sur au moins une partie de la hauteur de dents (h).

6. Procédé selon une des revendications 1 à 5, caractérisé en ce qu'on utilise des pièces de forme en forme de roue dentée dont les arêtes des têtes des dents sont chanfreinées ou arrondies.

7. Procédé selon une des revendications 1 à 6, caractérisé en ce que le catalyseur est composé d'une masse active (catalyseur plein) ou est revêtu d'une masse active (catalyseur sur support).

8. Procédé selon une des revendications 1 à 7, caractérisé en ce qu'on désydrogène de l'éthylbenzène pour obtenir du styrène.

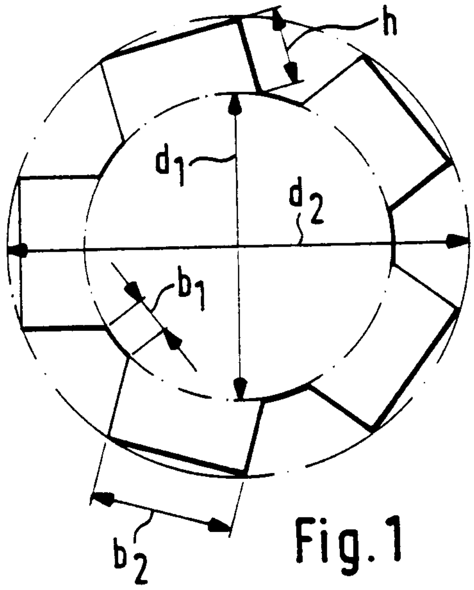


Fig. 1

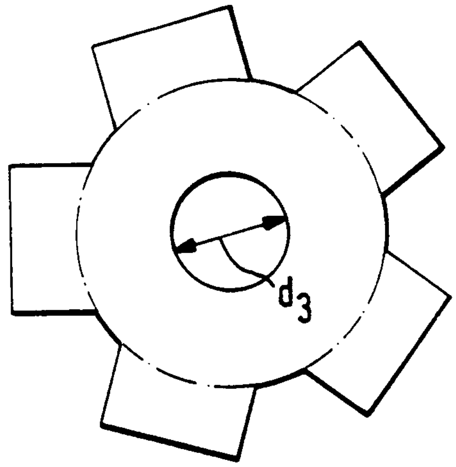


Fig. 2

Fig. 3

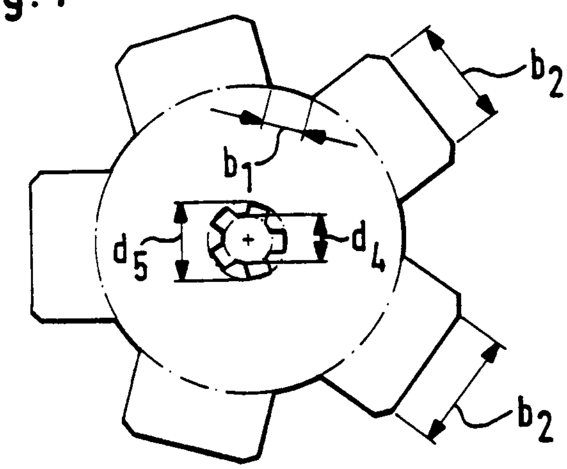


Fig. 4

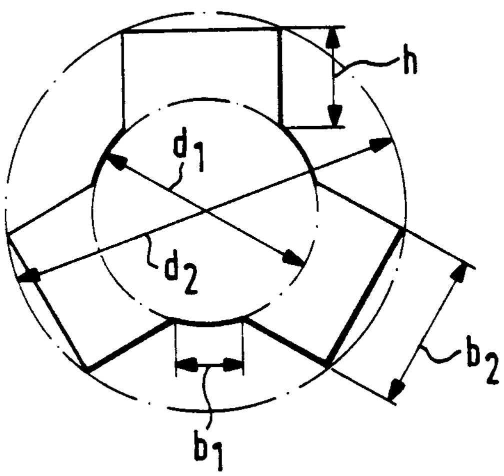


Fig. 5

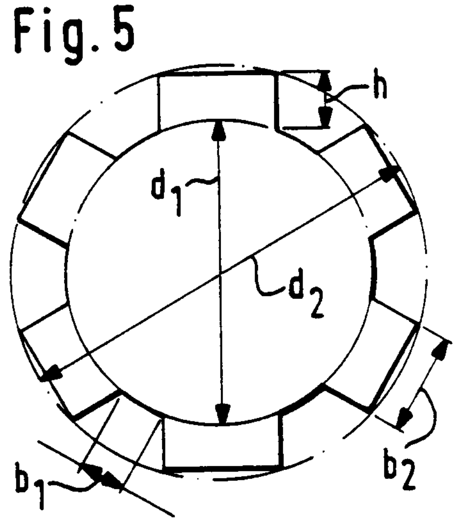

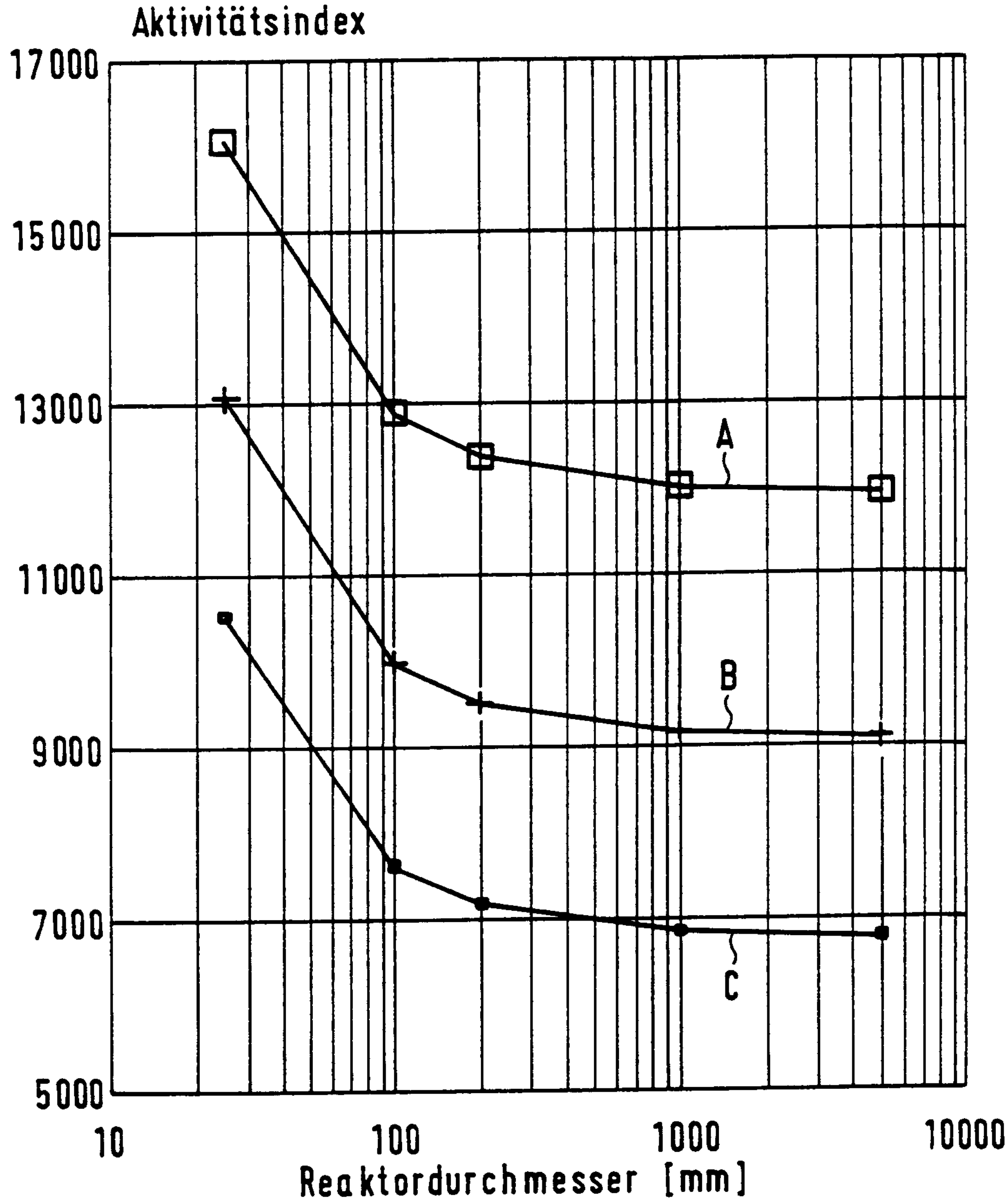
Aktivitätsindex
17000
15000
13000
11000
9000
7000
5000
10
100
1000
10000
Reaktordurchmesser [mm]
A
B
C

Fig. 6

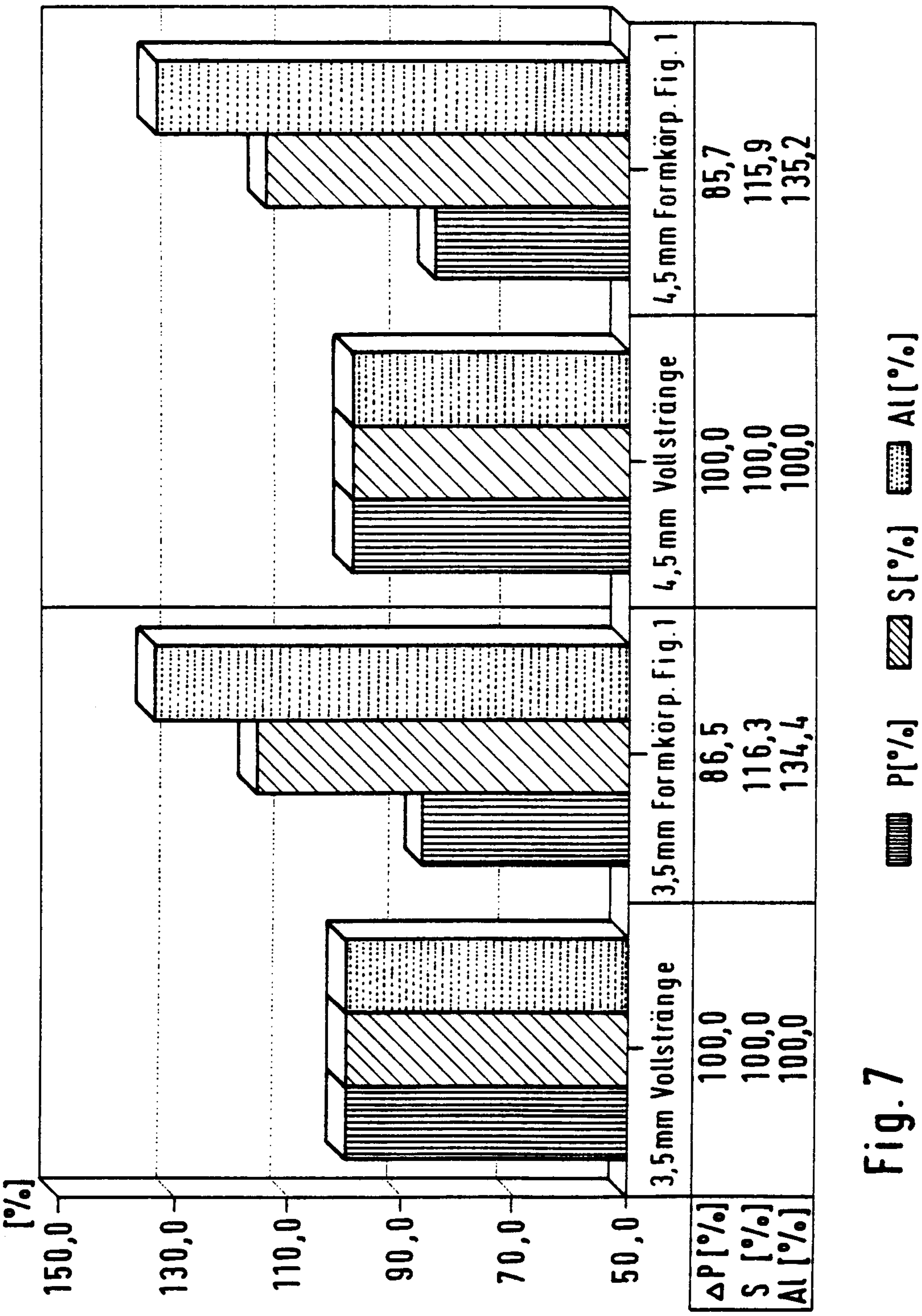
[%]
150,0
130,0
110,0
90,0
70,0
50,0
ΔP [%]
S [%]
Al [%]
3,5mm Vollstränge
100,0
100,0
100,0
3,5mm Formkörp. Fig. 1
86,5
116,3
134,4
4,5mm Vollstränge
100,0
100,0
100,0
4,5mm Formkörp. Fig. 1
85,7
115,9
135,2
P[%]
S[%]
Al[%]

Fig. 7

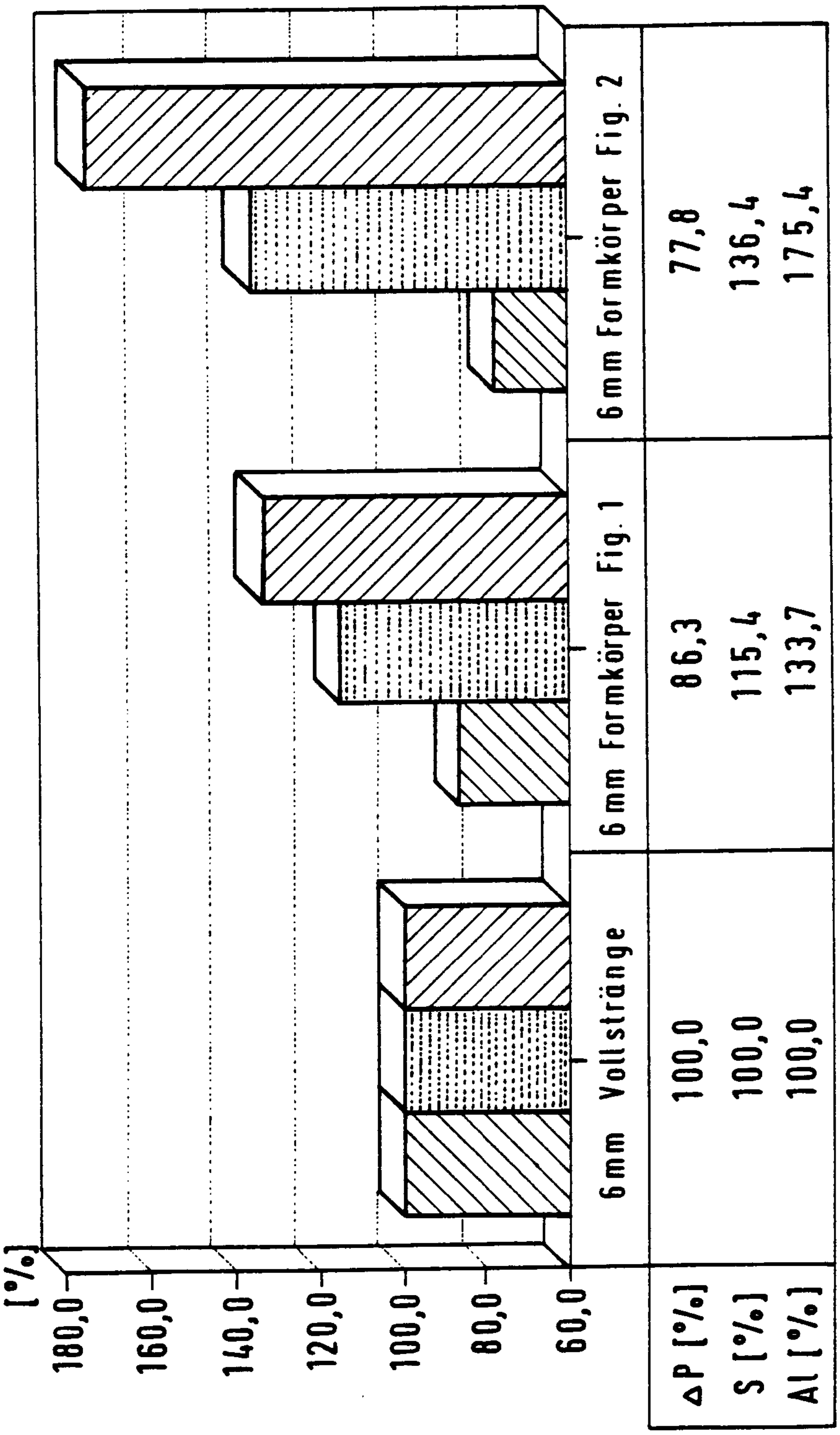
[%]
180,0
160,0
140,0
120,0
100,0
80,0
60,0
6mm Vollstränge
6mm Formkörper Fig. 1
6mm Formkörper Fig. 2
ΔP [%] 100,0 86,3 77,8
S [%] 100,0 115,4 136,4
Al [%] 100,0 133,7 175,4
ΔP[%]
S [%]
Al [%]

Fig. 8

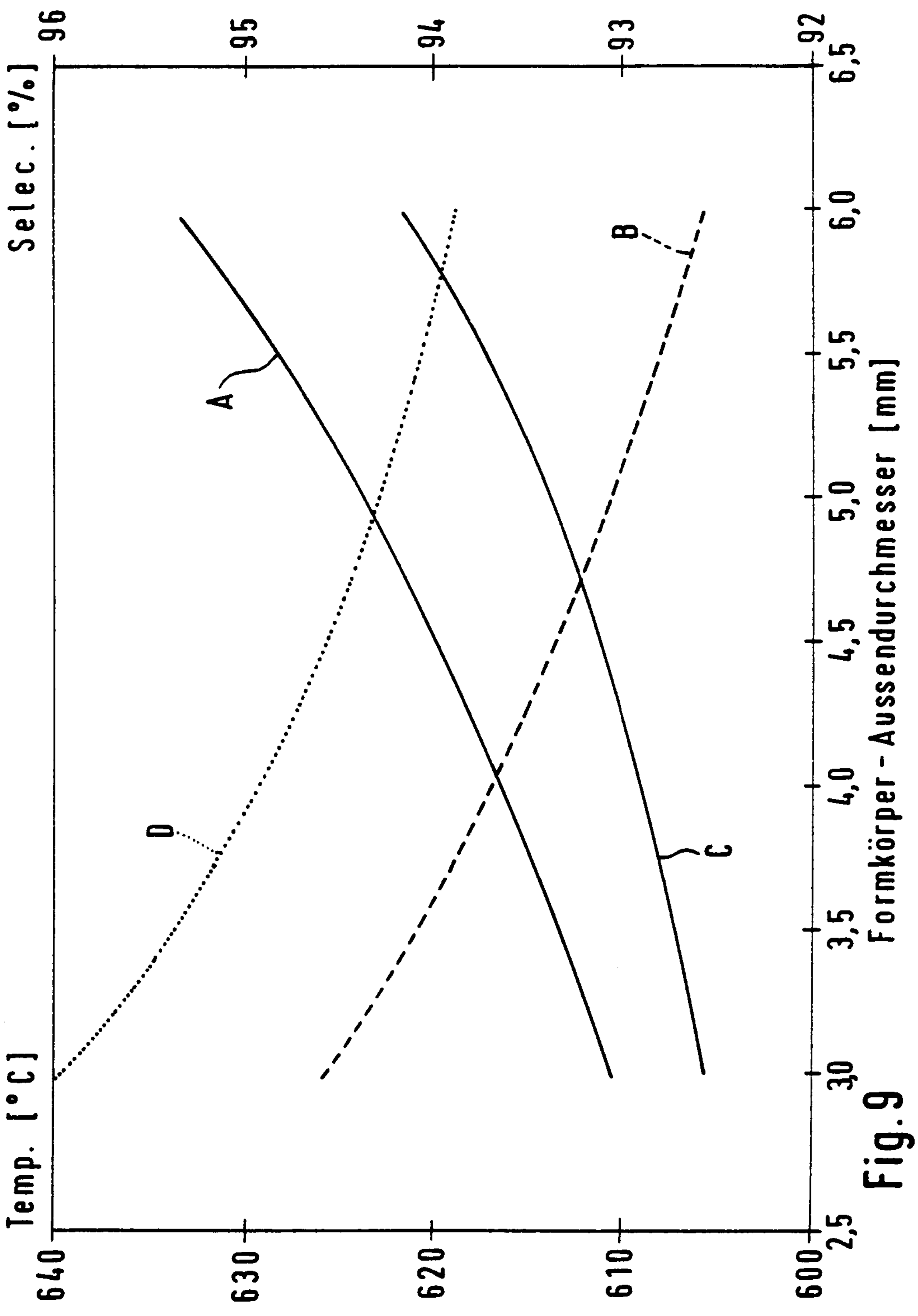

Temp. [°C]
Selec. [%]
640
630
620
610
600
96
95
94
93
92
2,5
3,0
3,5
4,0
4,5
5,0
5,5
6,0
6,5
A
B
C
D
Formkörper-Aussendurchmesser [mm]


Fig. 9